# EUROPÄISCHE PATENTSCHRIFT

(11) **EP 1 589 910 B1**
(45) Veröffentlichungstag und Bekanntmachung des Hinweises auf die Patenterteilung: **18.07.2007**
(21) Anmeldenummer: 03782406.7
(22) Anmeldetag: 15.12.2003
(51) Int. Cl.: A61F 5/01

(54) **SPRUNGGELENKORTHESE**
ANKLE JOINT ORTHESIS
ORTHESE DE L'ARTICULATION DE LA CHEVILLE

(30) Priorität: 28.01.2003 DE 10303326
(43) Veröffentlichungstag der Anmeldung: 02.11.2005
(73) Patentinhaber: OPED AG, 6312 Steinhausen (CH)
(72) Erfinder: HASSLER, Andreas, 83101 Rohrdorf (DE); HOPMANN, Gero, 85579 Neubiberg (DE)
(74) Vertreter: Tappe, Hartmut
(86) Internationale Anmeldenummer: PCT/EP2003/014251
(87) Internationale Veröffentlichungsnummer: WO 2004/066889

(56) Entgegenhaltungen:
- WO-A-02/051343
- US-A- 6 053 884

## Beschreibung

Die vorliegende Erfindung betrifft eine Sprunggelenkorthese zur äußeren Fixierung des Sprunggelenks mit einer medialen Stützschale und einer lateralen Stützschale, die mittels einer Verbundeinrichtung einen Schalenverbund bilden, der mit einer Auskleidung zur formschlüssigen Anlage der Sprunggelenkorthese versehen ist.

Sprunggelenkorthesen der eingangs genannten Art werden insbesondere zur Behandlung von Bänderdehnungen im Sprunggelenkbereich oder auch, insbesondere im Rahmen einer frühfunktionellen Therapie, nach operativen Eingriffen im Sprunggelenkbereich eingesetzt.

Eine entsprechende Sprunggelenksorthese ist aus der US-Patentschrift US-A-6, 053, 884 bekannt.

Unabhängig von der speziellen Indikation ist für eine optimale Wirkungsweise der Sprunggelenkorthese ein exakter und möglichst genau reproduzierbarer Sitz der Sprunggelenkorthese wesentlich. Darüber hinaus sollte eine Sprunggelenkorthese - insbesondere dann, wenn sie im Rahmen einer frühfunktionellen Therapie verwendet wird - vom Patienten selbst möglichst einfach zu handhaben sein, um einen möglichst hohen Grad an Akzeptanz und damit eine entsprechend häufige Benutzung der Sprunggelenkorthese erreichen zu können.

Der Erfindung liegt daher die Aufgabe zugrunde, eine Sprunggelenkorthese vorzuschlagen, die bei möglichst einfacher Handhabung eine möglichst genau reproduzierbare Positionierung der Sprunggelenkorthese mit optimaler Stützwirkung ermöglicht.

Diese Aufgabe wird durch eine Sprunggelenkorthese mit den Merkmalen des Anspruchs 1 gelöst.

Bei der erfindungsgemäßen Sprunggelenkorthese weist die Verbundeinrichtung eine formelastische Verbindungsspange mit tibiarer Spangenöffnung auf, die die Stützschalen in ihrer Relativanordnung veränderbar fixiert.

Mittels der formelastisch ausgebildeten Verbindungsspange sind die Stützschalen bereits vor,dem Anlegen der Sprunggelenkorthese in ihrer Relativanordnung definiert. Beim Anlegen, das durch ein fibulares Aufschieben der Sprunggelenkorthese auf das Sprunggelenk erfolgt, ermöglicht die formelastische Verbindungsspange ein Aufspreizen der Sprunggelenkorthese mit anschließender Überführung der Stützschalen in eine im Wesentlichen an den Sprunggelenkbereich beidseitig anliegende Grundkonfiguration der Sprunggelenkorthese aufgrund der formelastischen Rückstellkräfte. Darüber hinaus ermöglicht die veränderbare Relativpositionierung der Stützschalen zueinander eine individuelle Anpassung der Stützschalenpositionierung, so dass der Sitz der Sprunggelenkorthese den jeweiligen anatomischen Besonderheiten leicht angepasst werden kann.

Eine besonders komfortable Anpassung der Relativpositionierung der Stützschalen wird möglich, wenn die Verbindungsspange entsprechend einer bevorzugten Ausführungsform sowohl in ihrer Relativposition gegenüber der medialen Stützschale als auch in ihrer Relativposition gegenüber der lateralen Stützschale veränderbar ist.

Die Veränderung der Relativpositionierung wird besonders leicht und darüber hinaus einfach reproduzierbar möglich, wenn zur Verbindung der Verbindungsspange mit zumindest einer Stützschale in einem Verbindungsbereich der Stützschale eine Rastverbindungseinrichtung vorgesehen ist.

Eine einfache Betätigung der Rastverbindungseinrichtung durch fersenseitigen Druck auf die Verbindungsspange wird möglich, wenn die Rastverbindungseinrichtung mit einer quer zur Längserstreckung der Stützschalen angeordneten Führungseinrichtung versehen ist, die eine Verrastung von Spangenschenkeln der Verbindungsspange mit einer an den Stützschalen angeordneten Rasteinrichtung in mehreren Relativpositionen längs einer durch die Führungseinrichtung definierten Führungsbahn ermöglicht.

Eine fertigungstechnisch besonders leicht ausführbare und darüber hinaus die möglichst formsteife Ausgestaltung der Stützschalen fördernde Ausführungsform der Sprunggelenkorthese sieht vor, dass die Führungseinrichtung eine Führungssteganordnung mit zwei Führungsstegen zur zwischenliegenden Aufnahme eines Spangenschenkels der Verbindungsspange aufweist, wobei die Führungsstege zumindest abschnittsweise mit einem einen Randbereich des Spangenschenkels überdeckenden Vorsprung versehen sind.

Wenn die Führungseinrichtung eine Anschlageinrichtung zur Begrenzung einer Relativbewegung der Verbindungsspange längs der Führungsbahn aufweist, können die Extremstellungen der Relativpositionierung der Stützschalen in jedem Fall so vorgegeben werden, dass ein Anlegen der Sprunggelenkorthese unabhängig von der eingestellten Relativpositionierung möglich ist.

Wenn zur Ausbildung der Anschlageinrichtung zwischen den Führungsstegen der Stützschalen ein Anschlag vorgesehen ist, der mit zwei Querrändern einer in den Spangenschenkeln der Verbindungsspange ausgebildeten Anschlagnut zusammenwirkt, ist es möglich, die Relativstellung des Anschlags in der Anschlagnut gleichzeitig als Anzeigeeinrichtung für die Relativpositionierung der Stützschalen zueinander zu verwenden, um die Reproduzierbarkeit einer einmal ausgewählten Positionierung der Sprunggelenkorthese auch bei einem erneuten Anlegen derselben zu erleichtern.

Wenn die Stützschalen in einem Sprunggelenkbereich unterhalb des Verbindungsbereichs mit einer Ausnehmung für einen Knöchelbereich der Auskleidung versehen sind, ist zum einen eine besonders gute Formanpassung der Sprunggelenkorthese an den Sprunggelenkbereich möglich, zum anderen wird die Positionierung der Sprunggelenkorthese für einen optimalen Sitz derselben im Sprunggelenkbereich erleichtert. Darüber hinaus wird hierdurch ein Eingriff der Auskleidung in die Stützschalen ermöglicht, so dass eine Fixierung des Sitzes der Auskleidung in den Stützschalen die Folge ist.

Eine weitere Verbesserung der Fixierung der Auskleidung in den Stützschalen wird möglich, wenn die Stützschalen in einem Wadenbereich oberhalb des Verbindungsbereichs mit einer Ausnehmung für einen Formvorsprung der Auskleidung versehen sind.

Im Bereich der Verbindungsspange kann eine Fixierung der Relativpositionierung zwischen Auskleidung und Stützschalenverbund dadurch erfolgen, dass die Verbindungsspange mit einer Ausnehmung zur Durchführung eines Formvorsprungs der Auskleidung versehen ist.

Wenn die Auskleidung als ein mit Luft befüllbares oder evakuierbares Formkissen ausgebildet ist, kann der Formvorsprung durch eine Ventileinrichtung gebildet sein.

Wenn die vorstehend bereits erwähnten formelastischen Rückstellkräfte der Verbindungsspange zur Erzeugung der erforderlichen Stützwirkung der Sprunggelenkorthese nicht ausreichend sind, ist es möglich, die Verbindungsspange mit einer Verschlusseinrichtung zu versehen, die zur zugfesten Verbindung der Spangenschenkel über die tibiare Spangenöffnung geführt ist.

Darüber hinaus erweist es sich auch als vorteilhaft, zur zugfesten Verbindung der fußsohlenseitigen Längsenden der Stützschalen ein Stegband vorzusehen, das unter der Fußsohle längsgeführt werden kann, um insbesondere eine unerwünschte Aufwärtsbewegung der Sprunggelenkorthese - beispielsweise induziert durch Gehbewegungen - zu verhindern.

Wenn beide Längsenden des Stegbands jeweils über eine Klettverbindung mit den Stützschalen verbunden sind, ist eine Anpassung der wirksamen Stegbandlänge je nach Belieben des Patienten in besonders komfortabler Art und Weise mit der linken oder rechten Hand möglich.

Darüber hinaus ist es auch möglich, zur zugfesten Verbindung der Wadenbereiche der Stützschalen ein Wadenband vorzusehen, um auch im Wadenbereich einen besonders sicheren Sitz der Sprunggelenkorthese durch eine Stabilisierung des Stützschalenverbunds herbeizuführen.

Ein weiterer Beitrag zur sicheren Positionierung der Sprunggelenkorthese im Sprunggelenkbereich wird dadurch erreicht, dass die Auskleidung als Vakuumkissen ausgebildet ist, mit einer in einer Kissenhülle angeordneten Formkörperfüllung und einem einstückig mit dem Knöchelbereich des Vakuumkissens verbundenen, jedoch fluidtechnisch unabhängigen Polsterrand mit Fluidfüllung.

Als besonders vorteilhaft für die Positionierung der Auskleidung im Stützschalenverbund erweist es sich, wenn die Auskleidung drei fluidtechnisch miteinander verbundene Kissenteile aufweist, nämlich einen U-förmigen Verbindungsteil mit der in einem Basisbereich des Verbindungsteils angeordneten Ventileinrichtung und zwei jeweils in Längsrichtung von Schenkeln des Verbindungsteils verlaufende, von den freien Enden der Schenkel abgehende Schalenteile.

Eine bevorzugte Ausführungsform der Sprunggelenkorthese wird nachfolgend unter Bezugnahme auf die Zeichnungen_näher erläutert.

Es zeigen:
- **Fig. 1**: eine Sprunggelenkorthese mit Auskleidung in Seitenansicht mit Darstellung einer medialen Stützschale und einer die mediale Stützschale mit der in der Zeichnung nicht sichtbaren lateralen Stützschale verbindenden Verbindungsspange;
- **Fig. 2**: eine schienbeinseitige Vorderansicht der Sprunggelenkorthese;
- **Fig. 3**: eine Rückansicht der Sprunggelenkorthese mit Darstellung der die Stützschalen miteinander verbindenden Verbindungsspange;
- **Fig. 4**: eine Darstellung der Unterschenkelkontaktseite der in der Ebene ausgebreiteten Auskleidung;
- **Fig. 5**: eine Darstellung der Stützschalenkontaktseite der in der Ebene ausgebreiteten Auskleidung;
- **Fig. 6**: eine Darstellung der Extremstellungen der Stützschalen.

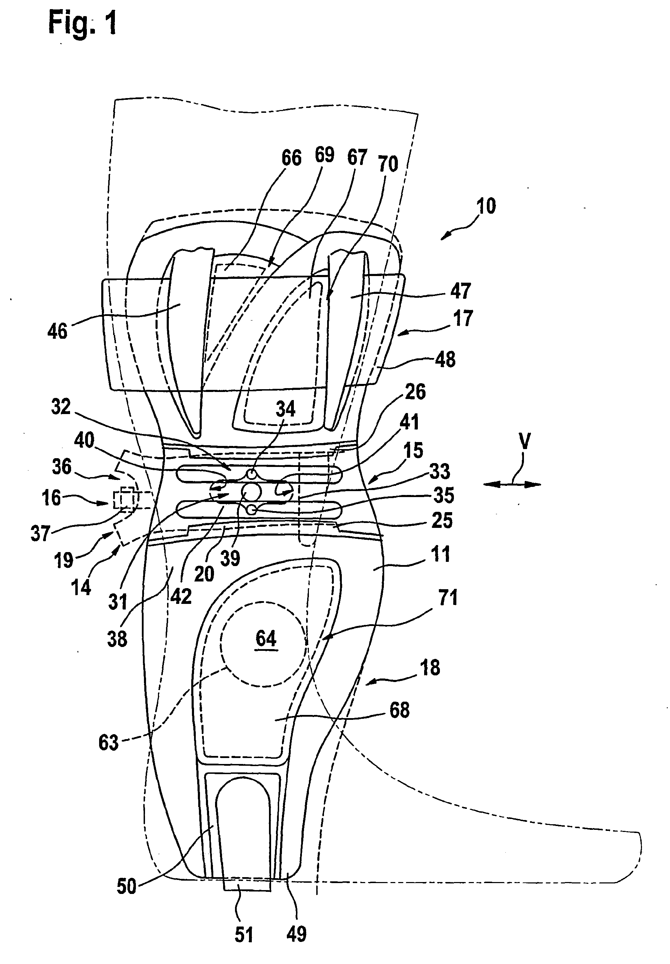

Aus einer Zusammenschau der **Fig. 1, 2** und **3** wird der Aufbau einer Sprunggelenkorthese 10 mit einer Verbindungsspange 14, die eine mediale Stützschale 11 und eine laterale Stützschale 12 zu einem Stützschalenverbund 13 vereinigt, deutlich. Die mediale Stützschale 11 und die laterale Stützschale 12 sind betreffend die nachfolgend erläuterten Merkmale übereinstimmend ausgebildet.

Wie **Fig. 1** zeigt, ist die mediale Stützschale 11 im Wesentlichen aus drei Abschnitten zusammengesetzt, nämlich einem Verbindungsbereich 15, der unter Mitwirkung der Verbindungsspange 14 zur Ausbildung einer Verbundeinrichtung 16 dient, einem oberhalb des Verbindungsbereichs 15 ausgebildeten Wadenbereich 17 und einem unterhalb des Verbindungsbereichs 15 ausgebildeten Sprunggelenkbereich 18.

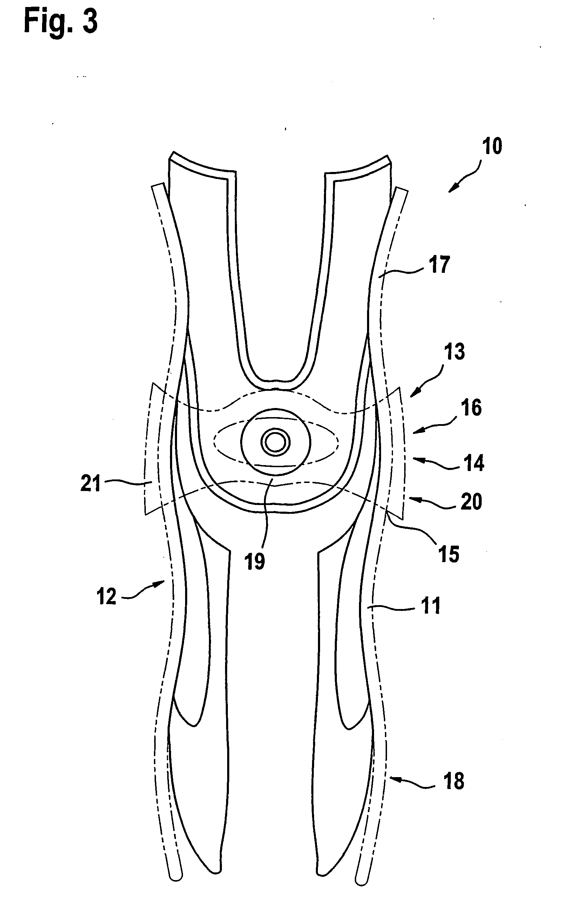

Wie insbesondere aus einer Zusammenschau der **Fig. 1** und **3** deutlich wird, ist die Verbindungsspange 14 im Wesentlichen U-förmig ausgeführt und umgreift mit ihren von einer Spangenbasis 19 abgehenden Spangenschenkeln 20, 21 jeweils eine zugeordnete Stützschale 11, 12. Um eine in Längsrichtung der Spangenschenkel 20, 21 bzw. quer zur Längserstreckung der Stützschalen 11, 12 veränderbare Fixierung der Spangenschenkel 20, 21 an den Stützschalen 11 bzw. 12 zu ermöglichen, ist die Verbundeinrichtung 16 bei dem vorliegenden Ausführungsbeispiel als Rastverbindung ausgelegt, die eine fixierte Relativpositionierung der Verbindungsspange 14 bzw. der Spangenschenkel 20, 21 gegenüber den Stützschalen 11, 12 ermöglicht.

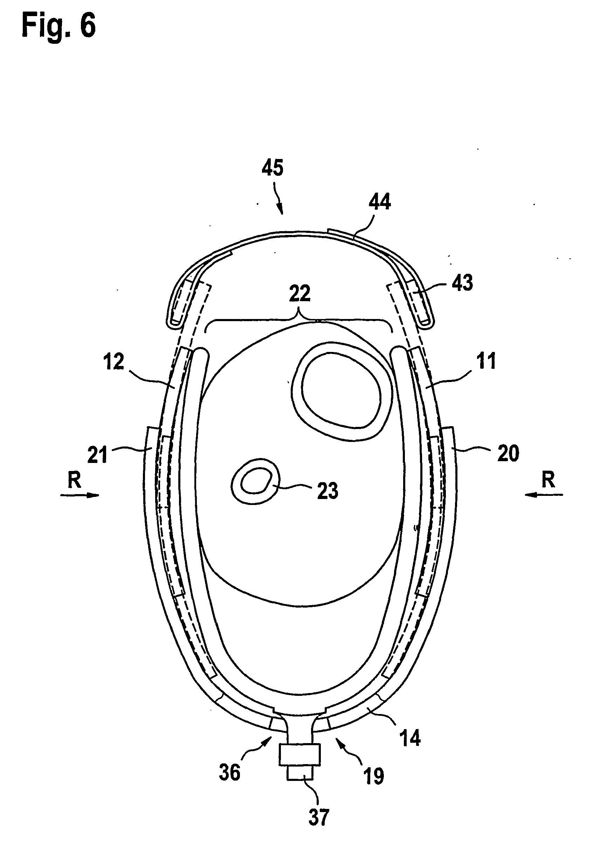

Zur Verdeutlichung der möglichen Relativpositionierungen sind in **Fig. 6** die den Verschiebeweg der Stützschalen 11, 12 gegenüber den Spangenschenkeln 20, 21 begrenzenden Extremstellungen der Stützschalen 11, 12 durch einen strichpunktierten bzw. gestrichelten Linienverlauf der Querschnittskonturen der Stützschalen 11, 12 angedeutet.

Die Verbindungsspange 14 ist beim vorliegenden Ausführungsbeispiel aus einem formelastischen Kunststoff, wie beispielsweise Polyamid gefertigt, so dass ein Auseinanderspreizen der Spangenschenkel 20, 21 entsprechende elastische Rückstellkräfte induziert, die in **Fig. 6** durch die Pfeile R angedeutet sind und ein Anpressen der Stützschalen 11, 12 gegen den Unterschenkel bewirken.

Ferner wird deutlich, dass das Aufspreizen der Spangenschenkel 20, 21 eine Erweiterung einer durch die freien Enden der Spangenschenkel 20, 21 begrenzten Spangenöffnung 22 ermöglicht und somit ein ungehindertes Aufschieben der Sprunggelenkorthese 10 bzw. des aus den Stützschalen 11, 12 gebildeten Stützschalenverbunds 13.

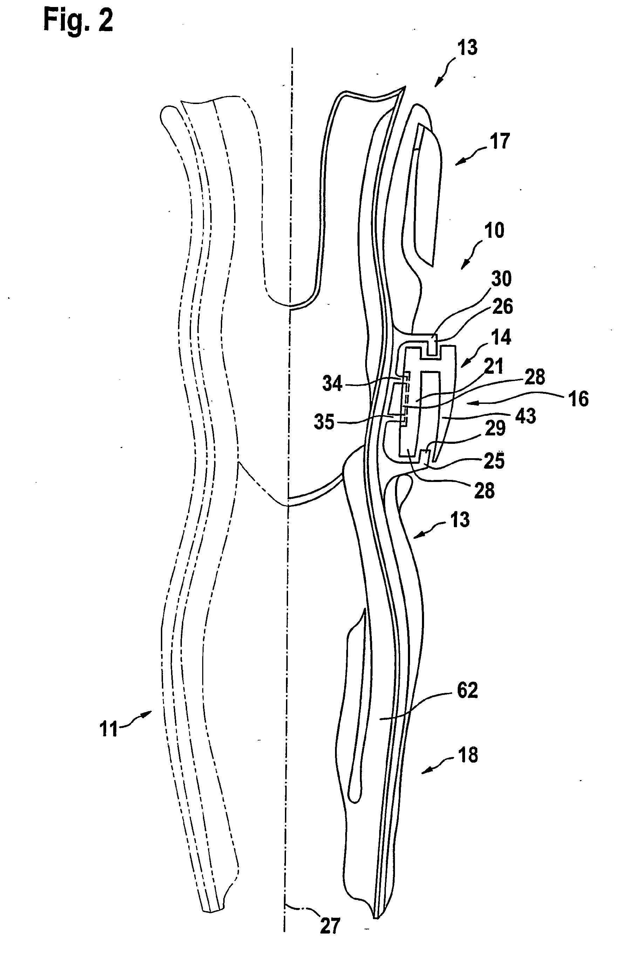

Wie insbesondere den **Fig. 1** und **2** zu entnehmen ist, umfasst die Verbundeinrichtung 16 eine im Verbindungsbereich 15 der Stützschalen 11, 12 ausgebildete Führungssteganordnung 24 mit zwei parallel zueinander angeordneten und quer zur Längsrichtung der Stützschale 11 verlaufenden Führungsstegen 25, 26. Zwischen den Führungsstegen 25, 26 ist ein Spangenschenkel 20 bzw. 21 geführt und wird zumindest abschnittsweise von als Haltestegen 29, 30 ausgebildeten Vorsprüngen übergriffen. Die Haltestege 29, 30 sind so dimensioniert, dass die aus formelastischem Material gefertigten Spangenschenkel hinter die Haltestege 29, 30 eingeclipst werden können, so dass eine Verbindung der Verbindungsspange 14 mit den Stützschalen 11, 12 sowohl leicht herstellbar als auch leicht lösbar ist, wobei gleichzeitig die zwischen der Verbindungsspange 14 und den Stützschalen 11, 12 hergestellte Verbindung ausreichend fest ist, um im Betrieb der Sprunggelenkorthese eine einwandfreie Funktion, also Fixierung der Stützschalen 11, 12 in ihrer Relativpositionierung durch die Verbindungsspange 14, zu ermöglichen.

Wie **Fig. 1** zeigt, ist zur Ausbildung einer Rastverbindungseinrichtung 31 zwischen dem Verbindungsbereich 15 der Stützschale 11 und dem Spangenschenkel 20 der Verbindungsspange 14 an der Stützschale 11 eine Rasteinrichtung 32 vorgesehen, die aus zwei seitlich an einem elastischen Steg 33 des Verbindungsbereichs 15 angeordneten Rastnasen 34, 35 besteht. Die Rastnasen 34, 35 greifen in auf einer Stützschalenkontaktseite 36 des Spangenschenkels 20 bzw. 21 ausgebildete quer zur Verschieberichtung V **(Fig. 1)** verlaufende Rastnuten 28 **(Fig. 2)** ein, die vorzugsweise äquidistant angeordnet sind und definierte Rastpositionen der Verbindungsspange 14 bzw. des Spangenschenkels 20, 21 gegenüber der Stützschale 11, 12 ermöglichen.

Wie ferner den **Fig. 1** und **6** zu entnehmen ist, ist die Spangenbasis 19 der Verbindungsspange 14 mit einer Durchgangsöffnung 36 versehen, die zur Durchführung einer Ventileinrichtung 37 der im vorliegenden Fall als evakuierbares Formkissen 38 ausgeführten Auskleidung dient. Die Anordnung der Ventileinrichtung 37 im Bereich der Durchgangsöffnung 36 der Spangenbasis 19 sorgt dabei nicht nur für eine geschützte Anordnung der Ventileinrichtung 37, sondern darüber hinaus für eine eindeutige Positionierung der Ventileinrichtung 37, die einen leichten Zugriff auf die Ventileinrichtung 37 zum Belüften bzw. Evakuieren des Formkissens 38 ermöglicht. Darüber hinaus ermöglicht die Anordnung der Ventileinrichtung 37 im Bereich der Durchgangsöffnung 36 auch eine Lagesicherung des Formkissens 38 gegenüber dem Stützschalenverbund 13 der Sprunggelenkorthese 10.

Zur Ausbildung definierter Endanschläge zwischen der Verbindungsspange 14 und den Stützschalen 11, 12, die den Verschiebeweg V in beide Richtungen begrenzen, ist die Stützschale 11 bzw. 12 auf dem Steg 33 des Verbindungsbereichs 15 mit einem Stoppnoppen 39 **(Fig. 1)** versehen, der in den Endstellungen des Verschiebeweges V gegen Querränder 40, 41 einer im Spangenschenkel 20 bzw. 21 ausgebildeten Anschlagnut 42 zum Anschlag kommt. Darüber hinaus kann die Relativstellung des von außen sichtbaren Stoppnoppens 39 gegenüber der Anschlagnut 42 als Anzeigeeinrichtung dienen, die die Reproduzierbarkeit einer gewählten Relativstellung zwischen der Verbindungsspange 14 und den Stützschalen 11, 12 erleichtert.

Insbesondere für den Fall, dass die durch die vorstehend erwähnten Rückstellkräfte bewirkte Anpresskraft der Stützschalen 11, 12 gegen den Unterschenkel des Patienten zur Erzielung einer sicheren Stützwirkung nicht ausreichend sein sollte, sind an den freien Enden der Spangenschenkel 20, 21 Zuglaschen 43 **(Fig. 6)** vorgesehen, um die Schlaufen eines beispielsweise über Klettverbindungen 44 gesicherten Zugbands 45 gelegt werden können, um durch Verkleinern der Spangenöffnungen 22 den Anpressdruck der Stützschalen 20, 21 gegen den Unterschenkel zu vergrößern.

Im Wadenbereich 17 der Stützschale 11 bzw. 12 **(Fig. 1)** sind im Fall des vorliegenden Ausführungsbeispiels zwei Führungslaschen 46, 47 vorgesehen, die nach Art von Gürtelschlaufen eine definierte Umschlingungsführung eines Wadenzugbands 48 um den Unterschenkel herum mit zwischenliegender Aufnahme der Wadenbereiche 17 der Stützschalen 11, 12 ermöglichen.

An unteren Längsenden 49 **(Fig. 1)** der Stützschalen 11, 12 sind vorzugsweise als Hakenfläche ausgebildete Befestigungsflächen 50 vorgesehen, die mit einer Schlingenfläche eines Stegbands 51 kontaktiert werden können, das sich zwischen den Befestigungsflächen 50 beider Stützschalen 11, 12 über die Fußsohle erstreckt und somit ein Hochrutschen der Sprunggelenkorthese 10 verhindert.

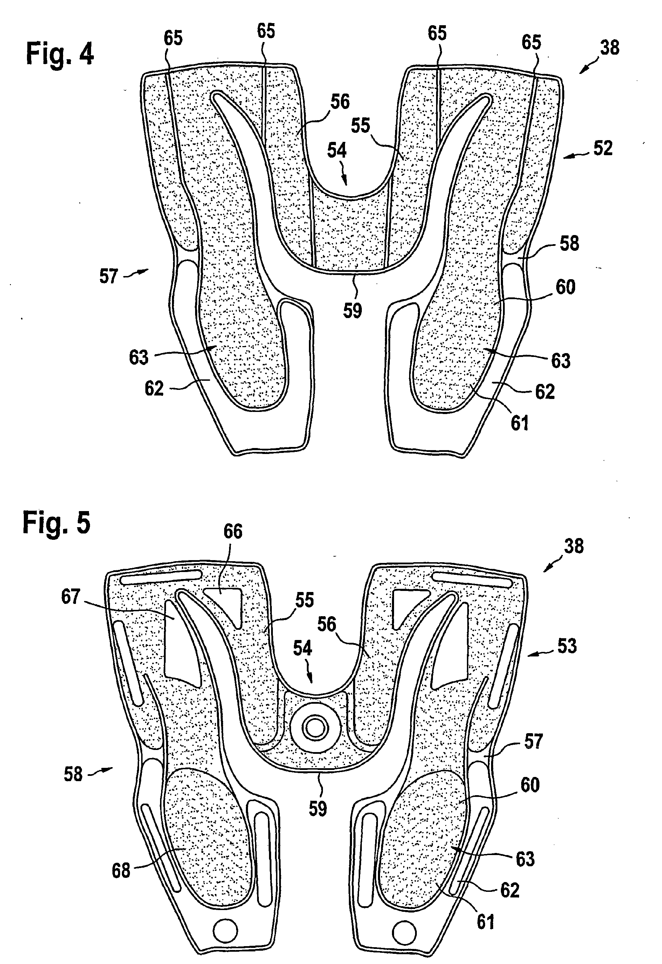

In den **Fig. 4** und **5** ist eine Draufsicht auf das in einer Ebene ausgebreitete Formkissen 38 dargestellt, wobei **Fig. 4** eine Unterschenkelkontaktseite 52 und **Fig. 5** eine Schalenkontaktseite 53 zeigt. Das Formkissen 38 weist drei ineinander übergehende Teile, nämlich einen mittleren, U-förmigen Verbindungsteil 54 und zwei jeweils am oberen Ende von Verbindungsteilschenkeln 55, 56 abgehende Schalenteile 57, 58, auf, die sich in etwa in Längsrichtung der Verbindungsteilschenkel 55, 56 bis unterhalb einer Verbindungsteilbasis 59 erstrecken. Die beiden Schalenteile 57, 58 des Formkissens 38 sind pneumatisch mit dem Verbindungsteil 54 verbunden und weisen wie dieser eine Formkörperfüllung 60 aus einzelnen Formkörpern 61 auf. Die Schalenteile 57, 58 weisen jeweils in einem unterhalb des Verbindungsteils 54 gelegenen Bereich längs ihres Außenrands verlaufend einen U-förmigen Polsterrand 62 auf, der fluidtechnisch von den übrigen Formkissenbereichen abgetrennt mit einem Luftvolumen befüllt ist. Der Polsterrand 62 umrandet einen Knöchelbereich 63 der Schalenteile 57, 58, der bei angelegter Sprunggelenkorthese 10, wie in **Fig. 1** dargestellt, auf dem Fußknöchel 64 zu liegen kommt.

Wie insbesondere aus der Darstellung gemäß **Fig. 2** hervorgeht, bildet der Polsterrand 62 zum einen eine Luftpolsterung, zum anderen sichert er durch die vom Polsterrand 62 umgebene Aufnahme des Knöchels 64 **(Fig. 1)** die Relativpositionierung der Stützschalen 11, 12 im Knöchelbereich.

Wie **Fig. 4** ferner zeigt, sind auf der Unterschenkelkontaktseite 52 des Formkissens 38 Scharnierbereiche 65 vorgesehen, in denen Querschnittsverengungen im Formkissen 38 ausgebildet sind, die einerseits immer noch einen ungestörten Fluiddurchtritt ermöglichen, andererseits jedoch im Durchmesser geschwächte Querschnittsbereiche zur Definition einer Scharnierfunktion ermöglichen. Aufgrund der Scharnierbereiche wird eine Anpassung des Formkissens 38 an den Stützschalenverbund 13 bzw. die Unterschenkelkontur erleichtert.

Wie **Fig. 5** zeigt, sind auf der Schalenkontaktseite 53 des Formkissens 38 sowohl im Wadenbereich des Formkissens 38 als auch im Knöchelbereich 63 gegenüber der Ebene der Schalenkontaktseite 53 erhabene Passformkörper 66, 67, 68 ausgebildet, die durch eine entsprechende Konturgebung der Schalenkontaktseite 53 gebildet sind und im Übrigen genau wie die umgebenden Bereiche des Formkissens 38 mit einer Formkörperfüllung 60 versehen sind. Wie **Fig. 1** zeigt, greifen die Passformkörper 66 und 67 in Passformausnehmungen 69, 70 ein, die im Wadenbereich 17 der Stützschalen 11, 12 angeordnet sind. Der Passformkörper 68 greift in eine entsprechend im Sprunggelenkbereich 18 der Stützschalen 11, 12 ausgebildete Passformausnehmung 71 ein. Hierdurch wird zum einen die relative Positionierung des Formkissens 38 gegenüber den Stützschalen 11, 12 gesichert. Zum anderen werden hierdurch Bereiche geschaffen, in denen eine Anpassung des Formkissens 38 infolge Evakuierung an die überdeckten Bereiche des Unterschenkels bzw. Sprunggelenks ohne eine Verformungsbehinderung durch die Stützschalen 11, 12 möglich ist.

## Patentansprüche

1. Sprunggelenkorthese zur äußeren Fixierung des Sprunggelenks mit einer medialen Stützschale und einer lateralen Stützschale, die mittels einer Verbundeinrichtung einen Schalenverbund bilden, der mit einer Auskleidung zur formschlüssigen Anlage der Sprunggelenkorthese versehen ist,
**dadurch gekennzeichnet,**
**dass** die Verbundeinrichtung (16) eine formelastische Verbindungsspange (14) mit tibiarer Spangenöffnung (22) aufweist, die die Stützschalen (11, 12) in ihrer Relativanordnung veränderbar fixiert.

2. Sprunggelenkorthese nach Anspruch 1,
**dadurch gekennzeichnet ,**
**dass** die Verbindungsspange (14) sowohl in ihrer Relativposition gegenüber der medialen Stützschale (11) als auch in ihrer Relativposition gegenüber der lateralen Stützschale (12) veränderbar ist.

3. Sprunggelenkorthese nach Anspruch 1 oder 2,
**dadurch gekennzeichnet ,**
**dass** zur Verbindung der Verbindungsspange (14) mit zumindest einer Stützschale (11, 12) in einem Verbindungsbereich (15) der Stützschale (11, 12) eine Rastverbindungseinrichtung (31) vorgesehen ist.

4. Sprunggelenkorthese nach Anspruch 3,
**dadurch gekennzeichnet ,**
**dass** die Rastverbindungseinrichtung (31) mit einer quer zur Längserstreckung der Stützschalen (11, 12) angeordneten Führungseinrichtung versehen ist, die eine Verrastung von Spangenschenkeln (20, 21) der Verbindungsspange (14) mit einer an den Stützschalen angeordneten Rasteinrichtung (32) in mehreren Relativpositionen längs einer durch die Führungseinrichtung definierten Führungsbahn ermöglicht.

5. Sprunggelenkorthese nach Anspruch 4,
**dadurch gekennzeichnet,**
**dass** die Führungseinrichtung eine Führungssteganordnung (24) mit zwei Führungsstegen (25, 26) zur zwischenliegenden Aufnahme eines Spangenschenkels (20, 21) der Verbindungsspange (14) aufweist, wobei die Führungsstege zumindest abschnittsweise mit einem einen Randbereich des Spangenschenkels überdeckenden Vorsprung (29, 30) versehen sind.

6. Sprunggelenkorthese nach einem der Ansprüche 4 oder 5,
**dadurch gekennzeichnet,**
**dass** die Führungseinrichtung mit einer Anschlageinrichtung zur Begrenzung einer Relativbewegung der Verbindungsspange (14) längs der Führungsbahn versehen ist.

7. Sprunggelenkorthese nach Anspruch 6,
**dadurch gekennzeichnet,**
**dass** zur Ausbildung der Anschlageinrichtung zwischen den Führungsstegen (25, 26) der Stützschalen (11, 12) ein Anschlag (33) vorgesehen ist, der mit zwei Querrändern (40, 41) einer in den Spangenschenkeln (20, 21) ausgebildeten Anschlagnut (42) zusammenwirkt.

8. Sprunggelenkorthese nach einem oder mehreren der vorangehenden Ansprüche,
**dadurch gekennzeichnet ,**
**dass** die Stützschalen (11, 12) in einem Sprunggelenkbereich (18) unterhalb des Verbindungsbereichs (15) mit einer Ausnehmung (71) für einen Knöchelbereich (68) der Auskleidung (38) versehen sind.

9. Sprunggelenkorthese nach einem oder mehreren der vorangehenden Ansprüche,
**dadurch gekennzeichnet,**
**dass** die Stützschalen (11, 12) in einem Wadenbereich (17) oberhalb des Verbindungsbereichs (15) mit einer Ausnehmung (69, 70) für einen Formvorsprung (66, 67) der Auskleidung (38) versehen sind.

10. Sprunggelenkorthese nach einem oder mehreren der vorangehenden Ansprüche,
**dadurch gekennzeichnet,**
**dass** die Verbindungsspange (14) mit einer Ausnehmung (36) zur Durchführung eines Formvorsprungs (37) der Auskleidung (38) versehen ist.

11. Sprunggelenkorthese nach Anspruch 10,
**dadurch gekennzeichnet,**
**dass** die Auskleidung (38) als ein mit Luft befüllbares oder evakuierbares Formkissen ausgebildet ist mit einer den Formvorsprung (37) bildenden Ventileinrichtung.

12. Sprunggelenkorthese nach einem oder mehreren der vorangehenden Ansprüche,
**dadurch gekennzeichnet ,**
**dass** die Verbindungsspange (14) eine Verschlusseinrichtung (43, 45) aufweist zur zugfesten Verbindung der Spangenschenkel (20, 21) über die tibiare Spangenöffnung (22).

13. Sprunggelenkorthese nach Anspruch 12,
**dadurch gekennzeichnet,**
**dass** zur zugfesten Verbindung der fußsohlenseitigen Längsenden (49) der Stützschalen (11, 12) ein Stegband (51) vorgesehen ist.

14. Sprunggelenkorthese nach Anspruch 13,
**dadurch gekennzeichnet ,**
**dass** beide Längsenden des Stegbands (51) jeweils über eine Klettverbindung (50) mit den Stützschalen (11, 12) verbunden sind.

15. Sprunggelenkorthese nach einem oder mehreren der vorangehenden Ansprüche,
**dadurch gekennzeichnet,**
**dass** zur zugfesten Verbindung der Wadenbereiche (17) der Stützschalen (11, 12) ein Wadenband (48) vorgesehen ist.

16. Sprunggelenkorthese nach einem oder mehreren der vorangehenden Ansprüche,
**dadurch gekennzeichnet,**
**dass** die Auskleidung (38) als Vakuumkissen ausgebildet ist mit einer in einer Kissenhülle angeordneten Formkörperfüllung (60) und einem einstückig mit jeweils einem Knöchelbereich (63) des Vakuumkissens fluidtechnisch unabhängig verbundenen Polsterrand (62) mit Fluidfüllung.

17. Sprunggelenkorthese nach einem oder mehreren der vorangehenden Ansprüche,
**dadurch gekennzeichnet,**
**dass** die Auskleidung (38) drei fluidtechnisch miteinander verbundene Kissenteile aufweist, nämlich einen U-förmigen Verbindungsteil (54) mit der in einem Basisbereich (59) des Verbindungsteils angeordneten Ventileinrichtung (37) und zwei jeweils in Längsrichtung von Schenkeln (55, 56) des Verbindungsteils verlaufende, von den freien Enden der Schenkel abgehende Schalenteile (57, 58).

## Claims

1. An orthotic device for the ankle joint for external fixation of the ankle joint, having a median supporting shell and a lateral supporting shell which form, by means of a joining device, a shell composite which is provided with a lining for form-fitting contact with the orthotic device for the ankle joint,
**characterized in that**
the composite device (16) has an elastic connecting clasp (14) with a tibial clasp opening (22) which secures the supporting shells (11, 12) in their relative arrangement to one another in a variable manner.

2. The orthotic device for the ankle joint according to Claim 1,
**characterized in that**
the connecting clasp (14) is variable in its relative position with respect to the median supporting shell (11) as well as in its relative position with respect to the lateral supporting shell (12).

3. The orthotic device for the ankle joint according to Claim 1 or 2,
**characterized in that**
for joining the connecting clasp (14) to at least one supporting shell (11, 12) in a connecting area (15) of the supporting shell (11, 12), a catch engagement device (31) is provided.

4. The orthotic device for the ankle joint according to Claim 3,
**characterized in that**
the catch engagement device (31) is provided with a guidance device arranged across the longitudinal extent of the supporting shells (11, 12), permitting a locking engagement of clasp legs (20, 21) of the connecting clasp (14) with a catch device (32) arranged on the supporting shells in several relative positions along a guidance path defined by the guidance device.

5. The orthotic device for the ankle joint according to Claim 4,
**characterized in that**
the guidance device has a guidance web arrangement (24) with two guidance webs (25, 26) for intermediate accommodation of a clasp leg (20, 21) of the connecting clasp (14), whereby the guidance webs are provided in at least some sections with a protrusion (29, 30) which covers an edge area of the clasp leg.

6. The orthotic device for the ankle joint according to any one of Claims 4 or 5,
**characterized in that**
the guidance device is provided with a stop device for limiting a relative movement of the connecting clasp (14) along the guidance path.

7. The orthotic device for the ankle joint according to Claim 6,
**characterized in that**
to form the stop device between the guidance webs (25, 26) of the supporting shells (11, 12), a stop (33) is provided, cooperating with two cross edges (40, 41) of a stop groove (42) provided in the clasp legs (20, 21).

8. The orthotic device for the ankle joint according to any one or more of the preceding claims,
**characterized in that**
the supporting shells (11, 12) are provided with a recess (71) for an ankle area (68) of the lining (38) in a talocrural area (18) below the connecting area (15).

9. The orthotic device for the ankle joint according to any one or more of the preceding claims,
**characterized in that**
the supporting shells (11, 12) are provided with a recess (69, 70) for a moulded protrusion (66, 67) of the lining (38) in a calf area (17) above the connecting area (15).

10. The orthotic device for the ankle joint according to any one or more of the preceding claims,
**characterized in that**
the connecting clasp (14) is provided with a recess (36) for allowing a mould protrusion (37) of the lining (38) to pass through.

11. The orthotic device for the ankle joint according to Claim 10,
**characterized in that**
the lining (38) is designed as a moulded cushion that can be filled with air or evacuated with a valve device which forms the mould protrusion (37).

12. The orthotic device for the ankle joint according to any one or more of the preceding claims,
**characterized in that**
the connecting clasp (14) has a locking device (43, 45) for connecting the clasp legs (20, 21) over the tibial clasp opening (22) in a connection that guarantees tensile strength.

13. The orthotic device for the ankle joint according to Claim 12,
**characterized in that**
a web band (51) is provided for connecting the longitudinal ends (49) of the supporting shells (11, 12) that are in the area of the sole of the foot in a connection that guarantees tensile strength.

14. The orthotic device for the ankle joint according to Claim 13,
**characterized in that**
the two longitudinal ends of the web band (51) are each connected to the supporting shells (11, 12) by a Velcro-type connection (50).

15. The orthotic device for the ankle joint according to any one or more of the preceding claims,
**characterized in that**
a calf band (48) is provided for a connection of the calf areas (17) of the supporting shells (11, 12) such that the connection guarantees tensile strength.

16. The orthotic device for the ankle joint according to any one or more of the preceding claims,
**characterized in that**
the lining (38) is designed as a vacuum cushion with a moulded body filling (60) arranged in a cushion cover and a padded edge (62), which is designed in one piece with an ankle area (63) of the vacuum cushion independently in fluid terms and has a fluid filling.

17. The orthotic device for the ankle joint according to any one or more of the preceding claims,
**characterized in that**
the lining (38) has three cushion parts joined together in fluid terms, namely a U-shaped connecting part (54) with a valve device (37) arranged in a base area (59) of the connecting part and with two shell parts (57, 58) branching off from the free ends of the legs, each running in the longitudinal direction of the legs (55, 56) of the connecting part.

## Revendications

1. - Orthèse de l'articulation de la cheville pour la fixation extérieure de l'articulation de la cheville comprenant une coquille d'appui médial et une coquille d'appui latéral, qui au moyen d'un dispositif de liaison forment une liaison de coquilles qui est pourvue d'une garniture pour l'installation de verrouillage par coopération de formes de l'orthèse de l'articulation de la cheville, **caractérisée en ce** le dispositif de liaison (16) présente une agrafe de liaison (14) de forme élastique comportant une ouverture d'agrafe (22) tibiale, qui fixe de façon modifiable les coquilles d'appui (11, 12) dans leur position relative.

2. - Orthèse de l'articulation de la cheville selon la revendication 1, **caractérisée en ce que** l'agrafe de liaison (14) est modifiable aussi bien dans sa position relative par rapport à la coquille d'appui médial (11) que dans sa position relative par rapport à la coquille d'appui latéral (12).

3. - Orthèse de l'articulation de la cheville selon la revendication 1 ou 2, **caractérisée en ce que** pour la liaison de l'agrafe de liaison (14) avec au moins une coquille d'appui (11, 12) il est prévu un dispositif de liaison par encliquetage (31) dans une zone de liaison (15) des coquilles d'appui (11, 12).

4. - Orthèse de l'articulation de la cheville selon la revendication 3, **caractérisée en ce que** le dispositif de liaison par encliquetage (31) comprend un dispositif de guidage s'étendant transversalement par rapport à l'extension longitudinale des coquilles d'appui (11, 12) qui permet un encliquetage d'ailes (20, 21) de l'agrafe de liaison (14) avec un organe d'encliquetage complémentaire (32) des coquilles d'appui dans plusieurs positions relatives le long d'un trajet de guidage défini par le dispositif de guidage.

5. - Orthèse de l'articulation de la cheville selon la revendication 4, **caractérisée en ce que** le dispositif de guidage comprend un agencement de chemins de guidage (24) avec deux chemins de guidage (25, 26) pour la réception entre eux d'une aile d'agrafe (20, 21) de l'agrafe de liaison (14), les chemins de guidage étant pourvus au moins sur des sections, d'une protubérance (29, 30) faisant saillie dans la zone du bord de l'aile d'agrafe.

6. - Orthèse de l'articulation de la cheville selon l'une des revendications 4 ou 5, **caractérisée en ce que** le dispositif de guidage comprend un agencement de butée pour limiter le déplacement relatif de l'agrafe de liaison (14) le long du trajet de guidage.

7. - Orthèse de l'articulation de la cheville selon la revendication 6, **caractérisée en ce que** pour réaliser l'agencement de butée entre les chemins de guidage (25, 26) des coquilles d'appui (11, 12) est prévu une butée (33) qui coopère avec deux bords transversaux (40, 41) d'une entaille d'appui (42) réalisée dans les ailes d'agrafe (20, 21).

8. - Orthèse de l'articulation de la cheville selon une ou plusieurs des revendications précédentes, **caractérisée en ce que** les coquilles d'appui (11, 12) sont pourvues dans une zone (18) de l'articulation de la cheville située en dessous de la zone de liaison (15) d'un évidement (71) pour une zone de cheville (68) de la garniture (38).

9. - Orthèse de l'articulation de la cheville selon une ou plusieurs des revendications précédentes, **caractérisée en ce que** les coquilles d'appui (11, 12) sont pourvues dans une zone de mollet (17) au dessus de la zone de liaison (15) avec un évidement (69, 70) pour une protubérance de forme (66, 67) de la garniture (38).

10. - Orthèse de l'articulation de la cheville selon une ou plusieurs des revendications précédentes, **caractérisée en ce que** l'agrafe de liaison (14) est pourvue d'un évidement (36) pour le passage d'une protubérance de forme (37) de la garniture (38).

11. - Orthèse de l'articulation de la cheville selon la revendication 10, **caractérisée en ce que** la garniture (38) est réalisé sous la forme d'un coussin pouvant être rempli ou vidé d'air avec un agencement de valve formant la protubérance de forme (37).

12. - Orthèse de l'articulation de la cheville selon une ou plusieurs des revendications précédentes, **caractérisée en ce que** l'agrafe de liaison (14) présente un dispositif de fermeture (43, 45) pour obtenir une liaison résistant à la traction des ailes de l'agrafe (20, 21) au dessus de l'ouverture tibiale (22) de l'agrafe.

13. **-** Orthèse de l'articulation de la cheville selon la revendication 12, **caractérisée en ce que** pour obtenir une liaison résistant à la traction, des coquilles d'appui (11, 12) une bande transversale (51) est prévue à l'extrémité de leur longueur (49) du côté de la plante du pied.

14. - Orthèse de l'articulation de la cheville selon la revendication 13, **caractérisée en ce que** les deux extrémités longitudinales de la bande transversale (51) sont chacune reliées avec les coquilles d'appui (11, 12) par une liaison agrippante (50).

15. - Orthèse de l'articulation de la cheville selon une ou plusieurs des revendications précédentes **caractérisée en ce que** pour obtenir une liaison résistante à la traction des zones de mollet (17) des coquilles d'appui (11, 12) une bande de mollet (48) est prévue.

16. - Orthèse de l'articulation de la cheville selon une ou plusieurs des revendications précédentes, **caractérisée en ce que** la garniture (38) est réalisée sous forme de coussin à vide comportant un corps de forme de remplissage (60) dans une enveloppe de coussin et une bordure rembourrée (62) réalisée d'une seule pièce avec une zone de cheville (63) du coussin à vide, cette bordure (62) étant reliée de façon fluidiquement indépendante et remplie de fluide.

17. - Orthèse de l'articulation de la cheville selon une ou plusieurs des revendications précédentes, **caractérisée en ce que** la garniture (38) présente trois parties de coussin reliées fluidiquement ensemble, à savoir une partie de liaison (54) en forme de U avec un dispositif de valve (37) disposé dans une zone de base (59) de la partie de liaison et deux parties de coquilles (57, 58) s'étendant chacune dans la direction longitudinale d'ailes (55, 56) de la partie de liaison, à partir des extrémités libres des ailes.
